(19) Europäisches Patentamt
European Patent Office
Office européen des brevets

(11) Veröffentlichungsnummer: **0 291 795**
**B1**

(12) # EUROPÄISCHE PATENTSCHRIFT

(45) Veröffentlichungstag der Patentschrift: **05.12.90**

(21) Anmeldenummer: **88107378.7**

(22) Anmeldetag: **07.05.88**

(51) Int. Cl.5: **C07D 249/08,** A01N 43/653, C07D 303/02, C07C 15/44, C07C 22/00, C07C 25/24, C07C 43/20, C07C 251/00

(54) **Hydroxyalkyl-triazolyl-Derivate.**

(30) Priorität: **18.05.87 DE 3716559**

(43) Veröffentlichungstag der Anmeldung: **23.11.88 Patentblatt 88/47**

(45) Bekanntmachung des Hinweises auf die Patenterteilung: **05.12.90 Patentblatt 90/49**

(84) Benannte Vertragsstaaten: **BE CH DE FR GB IT LI NL**

(56) Entgegenhaltungen:
**EP-A- 0 040 345**
**EP-A- 0 061 835**
**EP-A- 0 085 843**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Holmwood, Graham, Dr., Krutscheider Weg 105, D-5600 Wuppertal 11(DE)**
Erfinder: **Kraatz, Udo, Dr., Andreassstrasse 22a, D-5090 Leverkusen(DE)**
Erfinder: **Büchel, Karl Heinz, Prof. Dr., Dabringhausener Strasse 42, D-5093 Burscheid(DE)**
Erfinder: **Brandes, Wilhelm, Dr., Eichendorffstrasse 3, D-5653 Leichlingen(DE)**
Erfinder: **Dutzmann, Stefan, Dr., Leinenweberweg 33, D-4000 Düsseldorf 13(DE)**
Erfinder: **Reinecke, Paul, Dr., Steinstrasse 8, D-5090 Leverkusen 3(DE)**

**EP 0 291 795 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Hydroxyalkyltriazolyl-Derivate, ein Verfahren zu ihrer Herstellung sowie ihre Verwendung als Fungizide.

Es ist bereits bekannt, daß zahlreiche Hydroxyalkylazolyl-Derivate fungizide Eigenschaften besitzen (vgl. EP-A 0 040 345 und EP-A 0 061 835). So läßt sich z.B. 1-(4-Chlorphenyl)-4,4-dimethyl-3-(1,2,4-triazol-1-yl-methyl)-pentan-3-ol zur Bekämpfung von Pilzen einsetzen. Die Wirksamkeit dieses Stoffes ist sehr gut; allerdings läßt die Pflanzenverträglichkeit und die Wirksamkeit in manchen Fällen zu wünschen übrig.

Ferner sind aus der EP-A 0 085 843 Hydroxyethyl-azol-Derivate als Zwischenprodukte zur Herstellung von Antimykotika bekannt. Es werden aber keine Stoffe dieses Types beschrieben, in denen das zentrale Kohlenstoffatom andere Alkyl-Reste als tert.-Butyl trägt.

Es wurden nun neue Hydroxyalkyl-triazolyl-Derivate der Formel

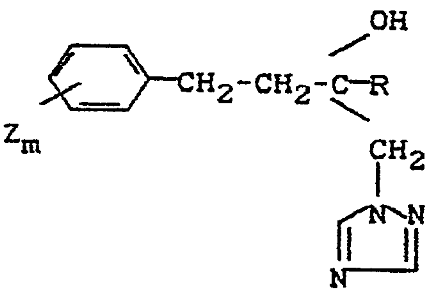

(I)

in welcher

R für die Reste der Formeln $-CH_2-CH(CH_3)_2$, $-(CH_2)_4-CH_3$, $-(CH_2)_5-CH_3$, $-(CH_2)_6-CH_3$,

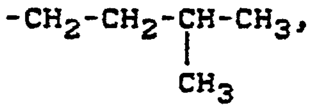

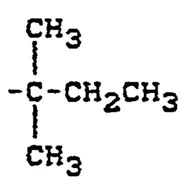 , 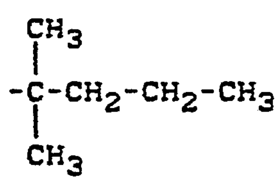 oder 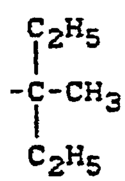 steht ,

steht ,

Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl oder Alkoximinomethyl mit 1 bis 4 Kohlenstoffatomen in der Alkoxygruppe steht und

m für die Zahlen 0, 1, 2 oder 3 steht,

sowie deren Säureadditions-Salze und Metallsalz-Komplexe gefunden.

Die neuen Hydroxyalkyl-triazolyl-Derivate der Formel (I) besitzen ein asymmetrisch substituiertes Kohlenstoffatom und können deshalb in den beiden optischen Isomerenformen anfallen. Die Erfindung betrifft sowohl die Racemate als auch die einzelnen Isomeren und deren Gemische.

Weiterhin wurde gefunden, daß man Hydroxyalkyl-triazolyl-Derivate der Formel (I) sowie deren Säureadditions-Salze und Metallsalz-Komplexe erhält, wenn man Oxirane der Formel

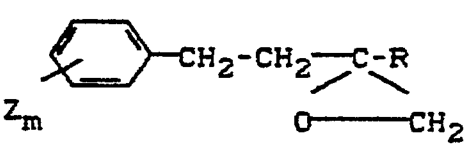

(II)

in welcher

R, Z und m die oben angegebene Bedeutung haben,

mit 1,2,4-Triazol der Formel

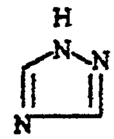

(III)

in Gegenwart eines Verdünnungsmittels und gegebenenfalls in Gegenwart eines Säurebindemittels sowie gegebenenfalls in Gegenwart eines Katalysators umsetzt und gegebenenfalls anschließend an die so erhaltenen Verbindungen der Formel (I) eine Säure oder ein Metallsalz addiert.

Schließlich wurde gefunden, daß sich die Hydroxyalkyl-triazolyl-Derivate der Formel (I) sowie deren Säureadditions-Salze und Metallsalz-Komplexe durch sehr gute fungizide Eigenschaften auszeichnen.

Überraschenderweise besitzen die erfindungsgemäßen Stoffe eine bessere fungizide Wirksamkeit als 1-(4-Chlorphenyl)-4,4-dimethyl-3-(1,2,4-triazol-1-yl-methyl)-pentan-3-ol, welches ein konstitutionell ähnlicher, vorbekannter Wirkstoff gleicher Wirkungsrichtung ist. Außerdem weisen die erfindungsgemäßen Stoffe eine hervorragende Pflanzenverträglichkeit auf.

Die erfindungsgemäßen Hydroxyalkyl-triazolyl-Derivate sind durch die Formel (I) allgemein definiert. Bevorzugt sind diejenigen Verbindungen der Formel (I), in denen

R für die Reste der Formeln $-CH_2-CH(CH_3)_2$, $-(CH_2)_4-CH_3$, $-(CH_2)_5-CH_3$, $-(CH_2)_6-CH_3$,

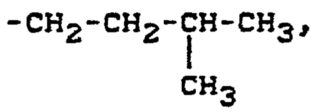

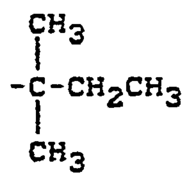 ,
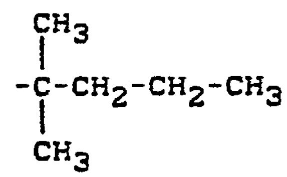 oder 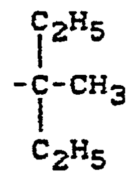 steht ,

steht

Z für Fluor, Chlor, Brom, Methyl, Ethyl, Trifluormethyl, Trifluormethoxy, Trifluormethylthio, Phenyl, Methoximinomethyl oder Ethoximinomethyl steht und

m für die Zahlen 0, 1, 2 oder 3 steht.

Wenn m für 2 oder 3 steht, können die Substituenten für Z gleich oder verschieden sein.

Bevorzugte erfindungsgemäße Verbindungen sind auch Additionsprodukte aus Säuren und denjenigen Hydroxyalkyl-triazolyl-Derivaten der Formel (I), in denen R, Z und m diejenigen Bedeutungen haben, die bereits im Zusammenhang mit der Beschreibung der erfindungsgemäßen Stoffe für diese Substituenten bzw. diesen Index als bevorzugt genannt wurden.

Zu den Säuren, die addiert werden können, gehören vorzugsweise Halogenwasserstoffsäure, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalin-disulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Hydroxyalkyl-triazolyl-Derivaten der Formel (I), in denen R, Z und m diejenigen Bedeutungen haben, die bereits im Zusammenhang mit der Beschreibung der erfindungsgemäßen Stoffe für diese Substituenten bzw. diesen Index als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzen-physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Verbindungen seien die in der folgenden Tabelle aufgeführten Stoffe genannt.

Tabelle

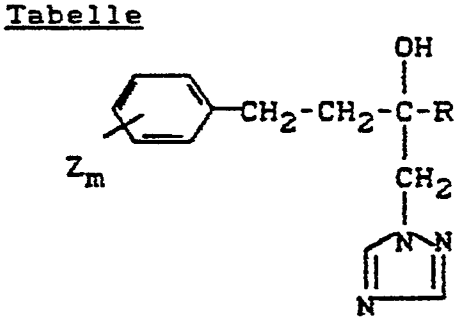

(I)

| $Z_m$ | R |
|---|---|
| 2,4-$Cl_2$ | $-(CH_2)_4-CH_3$ |
| 4-F | " |
| 4-CH=N-$OCH_3$ | " |
| 2,6-$Cl_2$ | " |

Tabelle (Fortsetzung)

| $Z_m$ | R |
|---|---|
| 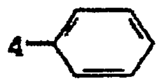 | " |
| - | " |
| $4-CF_3$ | " |
| $4-CH_3$ | " |
| $2,4-Cl_2$ | $-(CH_2)_5-CH_3$ |
| 4-F | " |
| $4-CH=N-OCH_3$ | " |
| $2,6-Cl_2$ | " |
| 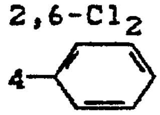 | " |
| - | " |
| $4-CF_3$ | " |
| $4-CH_3$ | " |
| $2,4-Cl_2$ | $-(CH_2)_6-CH_3$ |
| 4-F | " |
| $4-CH=N-OCH_3$ | " |
| $2,6-Cl_2$ | " |
| 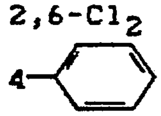 | " |
| - | " |
| $4-CF_3$ | " |
| $4-CH_3$ | " |

Tabelle (Fortsetzung)

| $Z_m$ | R |
|---|---|
| 2,4-$Cl_2$ | 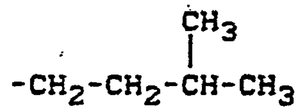 |
| 4-F | " |
| 4-CH=N-$OCH_3$ | " |
| 2,6-$Cl_2$ | " |
| 4-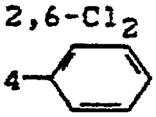 | " |
| - | " |
| 4-$CF_3$ | " |
| 4-$CH_3$ | " |
| 2,4-$Cl_2$ | 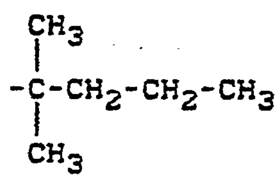 |
| 4-F | " |
| 4-CH=N-$OCH_3$ | " |
| 2,6-$Cl_2$ | " |
| 4-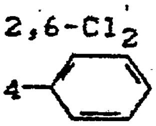 | " |
| - | " |
| 4-$CF_3$ | " |
| 4-$CH_3$ | " |

Verwendet man 2-[2-(4-Chlorphenyl)-ethyl]-2-[(1,1-dimethyl)-butyl]-oxiran und 1,2,4-Triazol als Ausgangssubstanzen, so kann der Verlauf des erfindungsgemäßen Verfahrens durch das folgende Formelschema wiedergegeben werden:

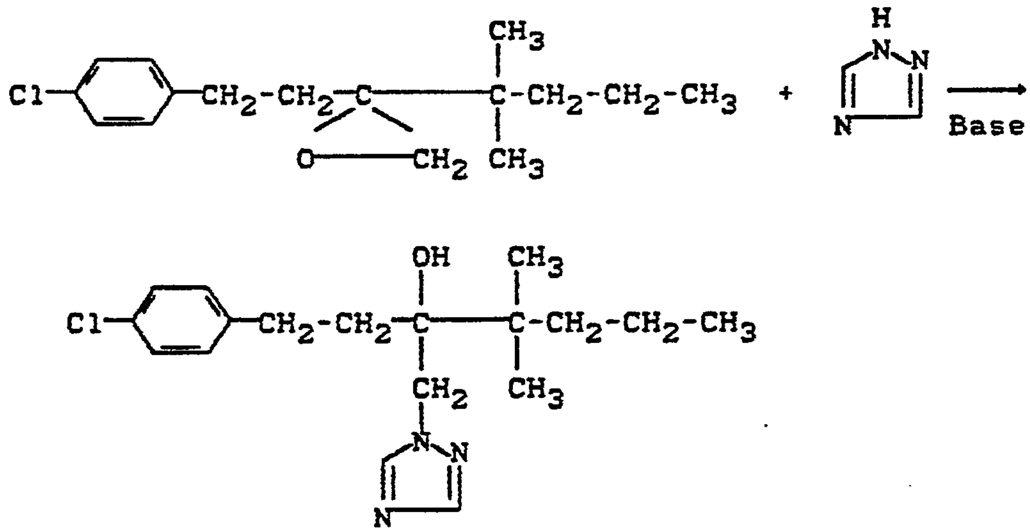


Die bei der Durchführung des erfindungsgemäßen Verfahrens als Ausgangsstoffe zu verwendenden Oxirane sind durch die Formel (II) allgemein definiert. In dieser Formel haben R, Z und der Index m vorzugsweise die Bedeutungen, die bereits im Zusammenhang mit der Beschreibung der erfindungsgemäßen Stoffe der Formel (I) für diese Substituenten bzw. für den Index m als bevorzugt genannt wurden.

Die Oxirane der Formel (II) sind noch nicht bekannt. Sie lassen sich herstellen, indem man

a) in einer <u>ersten Stufe</u> Ketone der Formel

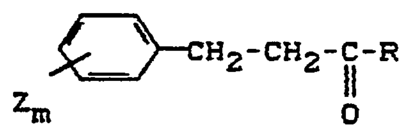
(IV)

in welcher
R, Z und m die oben angegebene Bedeutung haben,
mit Methyl-triphenyl-phosphoniumbromid der Formel

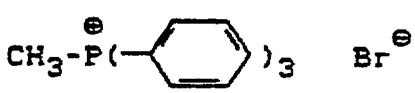
(V)

in Gegenwart einer Base und in Gegenwart eines Verdünnungsmittels umsetzt und dann in einer <u>zweiten Stufe</u> die so erhaltenen Verbindungen der Formel

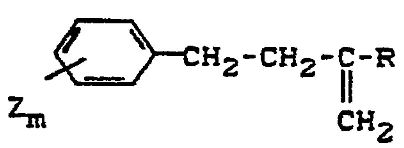
(VI)

in welcher
R, Z und m die oben angegebene Bedeutung haben,
mit Persäuren in Gegenwart eines Verdünnungsmittels umsetzt,
oder

b) Ketone der Formel

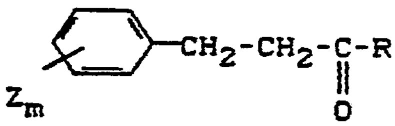

(IV)

in welcher
R, Z und m die oben angegebene Bedeutung haben, entweder
α) mit Dimethyloxosulfonium-methylid der Formel

$$\overset{\delta^{\ominus}}{(CH_3)_2SO}\ \overset{\delta^{\ominus}}{CH_2} \qquad (VII)$$

oder
β) mit Dimethylsulfonium-methylid der Formel

$$(CH_3)_2\overset{\delta^{\ominus}}{S}\ \overset{\delta^{\ominus}}{CH_2} \qquad (VIII)$$

in Gegenwart eines Verdünnungsmittels umsetzt.

Die bei der Herstellung der Oxirane der Formel (II) als Ausgangsstoffe benötigten Ketone der Formel (IV) sind bekannt oder lassen sich nach im Prinzip bekannten Verfahren in einfacher Weise herstellen (vgl. EP-OS 0 084 834). So erhält man Ketone der Formel (IV) dadurch, daß man Verbindungen der Formel

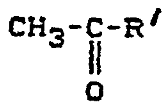

(IX)

in welcher
R′ die oben angegebene Bedeutung für R hat bzw. für entsprechende ungesättigte Substituenten von R steht,
mit Aldehyden der Formel

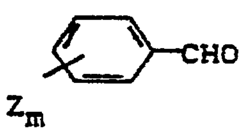

(X)

in welcher
Z und m die oben angegebene Bedeutung haben,
in Gegenwart eines Verdünnungsmittels, wie z.B. Ethanol, bei Temperaturen zwischen 0 und 60°C umsetzt und die dabei entstehenden Verbindungen der Formel

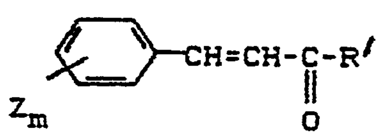

(XI)

in welcher
R′, Z und m die oben angegebene Bedeutung haben,
mit Wasserstoff in Gegenwart eines Katalysators, wie z.B. Raney-Nickel, in Gegenwart eines Verdünnungsmittels, wie z.B. Toluol oder Tetrahydrofuran, bei Temperaturen zwischen 40 und 180°C hydriert (vgl. auch die Herstellungsbeispiele).

Sowohl die Verbindungen der Formel (IX) als auch die Aldehyde der Formel (X) sind bekannt oder lassen sich nach bekannten Verfahren in einfacher Weise herstellen.

Das bei der Herstellung der Oxirane der Formel (II) nach dem Verfahren (a) weiterhin als Ausgangsmaterial benötigte Methyl-triphenyl-phosphonium-bromid der Formel (V) ist bekannt.

Die bei der Herstellung der Oxirane der Formel (II) nach dem obigen Verfahren (a) in der zweiten Stufe als Ausgangssubstanzen benötigten Verbindungen der Formel (VI) sind bisher noch nicht bekannt.

Bei dem Verfahren (a) zur Herstellung der Oxirane der Formel (II) arbeitet man in der ersten Stufe in Gegenwart einer Base. Dabei kommen als Basen alle üblicherweise für Wittig-Reaktionen dieser Art verwendbaren Base in Betracht. Vorzugsweise verwendbar ist Kalium-tert.-butylat.

Als Verdünnungsmittel kommen bei der Durchführung der ersten Stufe des obigen Verfahrens (a) zur Herstellung der Oxirane der Formel (II) alle für derartige Umsetzungen üblichen organischen Solventien infrage. Vorzugsweise verwendbar sind aromatische Kohlenwasserstoffe, wie Benzol, Toluol und Xylol.

Bei der Durchführung der zweiten Stufe des obigen Verfahrens (a) zur Herstellung der Oxirane der Formel (II) kommen als Reagenzien zur Epoxidierung alle üblichen Persäuren in Betracht. Vorzugsweise verwendbar sind meta-Chlorperbenzoesäure und Peressigsäure. Ferner ist es auch möglich, ein Gemisch aus Essigsäure und Wasserstoffperoxid einzusetzen.

Als Verdünnungsmittel kommen bei der Durchführung der zweiten Stufe des obigen Verfahrens (a) zur Herstellung der Oxirane der Formel (II) alle für derartige Epoxidierungen üblichen Solventien infrage. Vorzugsweise verwendbar sind Dichlormethan, Chloroform, Toluol, Dichlorbenzol, Essigsäure und andere inerte Solventien.

Die Reaktionstemperaturen können bei der Durchführung des Verfahrens (a) zur Herstellung der Oxirane der Formel (II) innerhalb eines bestimmten Bereiches variiert werden. Im allgemeinen arbeitet man in der ersten Stufe bei Temperaturen zwischen 50°C und 140°C, vorzugsweise zwischen 80°C und 120°C. In der zweiten Stufe arbeitet man im allgemeinen zwischen 10°C und 60°C, vorzugsweise zwischen 20°C und 50°C.

Bei der Durchführung des Verfahrens (a) zur Herstellung der Oxirane der Formel (II) geht man im allgemeinen so vor, daß man in der ersten Stufe auf 1 Mol an Keton der Formel (IV) zwischen 1 und 3 Mol Methyl-triphenylphosphonium-bromid der Formel (V) sowie zwischen 1 und 3 Mol Base einsetzt. In der zweiten Stufe setzt man auf 1 Mol an einer Verbindung der Formel (VI) jeweils zwischen 1 und 2 Mol an Persäure ein. Die Aufarbeitung erfolgt jeweils nach üblichen Methoden.

Das bei dem Verfahren (b) als Reaktionskomponente benötigte Dimethyloxosulfoniummethylid der Formel (VII) ist bekannt (vgl. J. Amer. Chem. Soc. 87, 1363-1364 (1965)). Es wird bei der obigen Umsetzung in frisch zubereitetem Zustand verarbeitet, indem man es in situ durch Umsetzung von Trimethyloxo-sulfonium-iodid mit Natriumhydrid oder Natriumamid, insbesondere mit Kalium-tert.-butylat oder Natriummethylat, in Gegenwart eines Verdünnungsmittels erzeugt.

Das bei dem Verfahren (b) außerdem als Reaktionskomponente in Betracht kommende Dimethylsulfonium-methylid der Formel (VIII) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfonium-halogenid oder Trimethylsulfonium-methylsulfat, in Gegenwart einer starken Base, wie zum Beispiel Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (b) inerte organische Solventien infrage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei dem Verfahren (b) in einem größeren Bereich variiert werden. Im allgemeinen arbeitet man bei Temperaturen zwischen 0 und 100°C, vorzugsweise zwischen 10 und 60°C.

Bei der Durchführung des Verfahrens (b) setzt man auf 1 Mol an Keton der Formel (IV), vorzugsweise 1 bis 3 Mol an Dimethyloxosulfonium-methylid der Formel (VII) bzw. an Dimethylsulfonium-methylid der Formel (VIII) ein. Die Isolierung der Oxirane erfolgt nach üblichen Methoden.

Die Oxirane der Formel (II) können bei dem erfindungsgemäßen Verfahren gegebenenfalls ohne Isolierung direkt weiter umgesetzt werden.

Als Verdünnungsmittel kommen für das erfindungsgemäße Verfahren unter den Reaktionsbedingungen inerte organische Lösungsmittel infrage. Vorzugsweise verwendbar sind Alkohole, wie z.B. Ethanol, Methoxyethanol oder Propanol; Ketone, wie z.B. 2-Butanon und N-Methyl-pyrrolidon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für das erfindungsgemäße Verfahren alle üblicherweise verwendbaren anorganischen und organischen Basen in Betracht. Vorzugsweise verwendbar sind Alkali metallcarbonate, wie z.B. Natrium- und Kaliumcarbonat; Alkalimetallhydroxide, wie z.B. Natriumhydroxid; Alkalimetallalkoholate, wie z.B. Natrium- und Kalium-methylat und -ethylat; Alkalimetallhydride, wie z.B. Natriumhydrid; sowie niedere tertiäre Alkylamine, Cycloalkylamine und Aralkylamine, wie insbesondere Triethylamin.

Als Katalysatoren kommen bei der Durchführung des erfindungsgemäßen Verfahrens alle üblicherweise für derartige Umsetzungen einsetzbaren Reaktionsbeschleuniger infrage. Vorzugsweise verwendbar ist $\alpha,\alpha'$-Azo-isobutyronitril.

Die Reaktionstemperaturen können bei der Durchführung des erfindungsgemäßen Verfahrens in einem größeren Bereich variiert werden. Im allgemeinen arbeitet man bei Temperaturen zwischen 0 und 200°C, vorzugsweise zwischen 60 und 150°C.

Bei der Durchführung des erfindungsgemäßen Verfahrens arbeitet man im allgemeinen unter Normaldruck. Es ist jedoch auch möglich, unter erhöhtem oder vermindertem Druck zu arbeiten.

Bei der Durchführung des erfindungsgemäßen Verfahrens setzt man auf 1 Mol Oxiran der Formel (II) vorzugsweise 1 bis 2 Mol an 1,2,4-Triazol und gegebenenfalls 1 bis 2 Mol an Säurebindemittel ein. Die Aufarbeitung und die Isolierung der Endprodukte erfolgt nach üblichen Methoden.

Die nach dem erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen infrage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Stoffe mit besonders gutem Erfolg zur Bekämpfung von Venturia (Apfel), Cercospora (Mungobohne) sowie von Erysiphe, Puccinia, Leptosphaeria, Cochliobolus und Pyrenophora an Getreide und von Pyricularia und Pellicularia an Reis eingesetzt werden.

Darüber hinaus besitzen die erfindungsgemäßen Verbindungen ein gutes, breites in-vitro-Wirkungsspektrum.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsgulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Stoffe geht aus den folgenden Beispielen hervor.

Herstellungsbeispiele

Beispiel 1

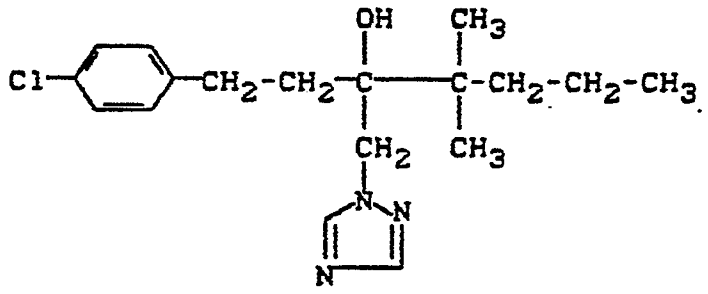

(I-1)

Eine Lösung von 20,5 g (0,077 Mol) 2-(4-Chlorphenylethyl)-2-(1,1-dimethylbutyl)-oxiran, 5,3 g (0,077 Mol) 1,2,4-Triazol, 1,0 g (0,025 Mol) Natriumhydroxid, 1 ml Wasser und einer Spatelspitze α,α'-Azoisobutyronitril in 50 ml N-Methylpyrrolidon wird 5 Stunden auf 120°C erwärmt. Danach kühlt man auf Raumtemperatur ab, engt durch Abziehen des Lösungsmittels unter vermindertem Druck ein, löst den verbleibenden Rückstand in Essigester, wäscht dreimal mit Wasser, trocknet die organische Phase über Natriumsulfat und entfernt das Lösungsmittel unter vermindertem Druck. Der Rückstand wird durch Säulenchromatographie (Kieselgel; Dichlormethan : Essigester = 4 : 1) gereinigt. Man erhält auf diese Weise 16,0 g (61,9 % der Theorie) an 1-(4-Chlorphenyl)-4,4-dimethyl-3-(1,2,4-triazol-1-yl-methyl)-3-heptanol in Form eines gelben Öls.

Die Substanz ist durch das in Abbildung 1 gezeigte Kernresonanzspektrum charakterisiert.

Herstellung von Ausgangsprodukten

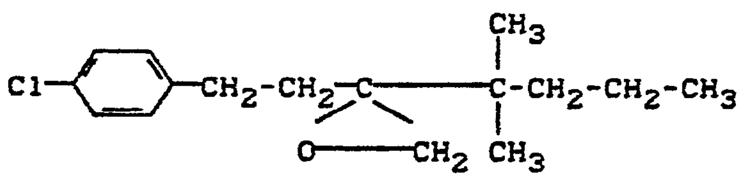

(II-1)

In eine siedende Lösung von 22 g (0,88 Mol) 2-(4-Chlorphenylethyl)-3,3-dimethyl-1-hexen in 100 ml Dichlormethan wird innerhalb von 2,5 Stunden eine Lösung von 18,2 g (0,106 Mol) m-Chlorperbenzosäure in 250 ml Dichlormethan eingetropft. Man erhitzt weitere 2 Stunden unter Rückfluß, kühlt dann auf Raumtemperatur ab, wäscht zunächst dreimal mit 1 N wäßriger Natronlauge und danach mit Wasser, trocknet dann die organische Phase über Natriumsulfat und engt durch Abziehen des Lösungsmittels unter vermindertem Druck ein. Man erhält 20,5 g eines farblosen Öls, das gemäß gaschromatographischer und massenspektrometrischer Analyse zu 89,6 % aus 2-(4-Chlorphenylethyl)-2-(1,1-dimethylbutyl)-oxiran besteht. Die Ausbeute errechnet sich danach zu 78,5 % der Theorie. Das Produkt wird ohne zusätzliche Reinigung für die weitere Umsetzung verwendet.

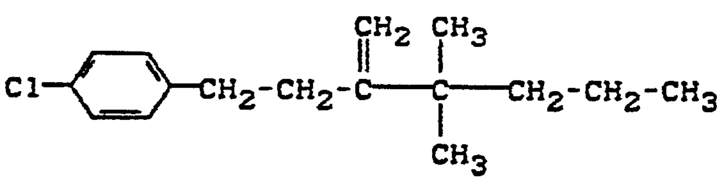

(VI-1)

Eine Suspension aus 47,4 g (0,133 Mol) Methyl-triphenylphosphoniumbromid und 15,3 g (0,137 Mol) Kalium-tert.-butylat in 250 ml absolutem Toluol wird unter trockenem Stickstoff 30 Minuten unter Rückfluß erhitzt. Innerhalb von 5 Minuten werden dann 25,3 g (0,1 Mol) 1-(4-Chlorphenyl)-4,4-dimethyl-3-heptanon, gelöst in 10 ml absolutem Toluol, eingetropft. Das Reaktionsgemisch wird weitere 15 Stunden unter Rückfluß erhitzt, dann auf Raumtemperatur abgekühlt, zweimal mit Wasser gewaschen und unter vermindertem Druck eingeengt. Man nimmt den Rückstand in Essigester auf, kühlt auf 5°C ab und saugt den gebildeten Kristallbrei ab. Das Filtrat wird eingeengt und unter vermindertem Druck destilliert. Man erhält 23 g (91,8 % der Theorie) an 2-(4-Chlorphenylethyl)-3,3-dimethyl-1-hexen in Form eines gelben Öls vom Siedepunkt 85-87°C/0,1 mbar.

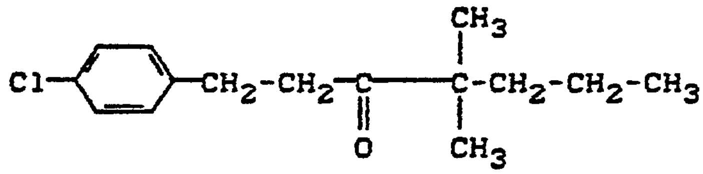

(IV-1)

Eine Lösung von 92 g (0,37 Mol) 1-(4-Chlorphenyl)-4,4-dimethyl-1,6-heptadien-3-on in 400 ml Tetrahydrofuran wird mit 15 g Raney-Nickel versetzt und im Autoklaven 2,5 Stunden bei 40°C unter einem Wasserstoffdruck von 50 bar gerührt. Danach wird das Reaktionsgemisch filtriert und unter vermindertem Druck eingeengt. Nach Destillation des Rückstands im Vakuum erhält man 55 g (58,9 % der Theorie) 1-(4-Chlorphenyl)-4,4-dimethyl-3-heptanon in Form eines gelben Öls vom Siedepunkt 179°C/16 mbar.

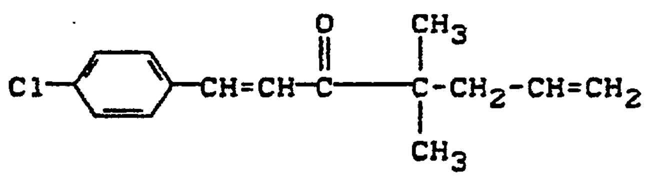

(XI-1)

Eine Lösung von 56,2 g (0,4 Mol) 4-Chlorbenzaldehyd und 50,4 g (0,4 Mol) 4,4-Dimethyl-1-hexen-5-on in 200 ml Ethanol wird mit 40 ml Wasser und direkt danach mit einer Lösung von 1,2 g Natriumhydroxid in 12 ml Wasser versetzt. Das Gemisch wird zunächst 1 Stunde bei Raumtemperatur gerührt, dann mit 0,8 g festem Natriumhydroxid versetzt und weitere 16 Stunden gerührt. Es wird mit Wasser verdünnt und mit Essigester extrahiert. Der Essigester-Extrakt wird dreimal mit Wasser gewaschen, getrocknet und eingeengt. Man erhält 92 g (92,5 % der Theorie) 1-(4-Chlorphenyl)-4,4-dimethyl-1,6-heptadien-3-on in Form eines gelben Öls.

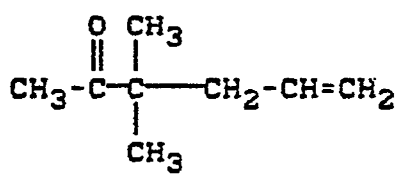

(IX-1)

Ein Gemisch von 121 g (1,0 Mol) 3-Brompropen und 103 g (1,2 Mol) 3-Methyl-2-butanon wird innerhalb von 2 Stunden in eine Suspension von 168 g (3,0 Mol) Kaliumhydroxid-Pulver und 10 g Tetrabutylammoniumbromid in 300 ml Toluol unter Rühren eingetropft. Dabei wird die Reaktionstemperatur unter 30°C gehalten. Das Reaktionsgemisch wird weitere 2 Stunden bei Raumtemperatur gehalten, dann mit Wasser versetzt, und die organische Phase wird abgetrennt. Die organische Phase wird zweimal mit Wasser gewaschen, getrocknet und über eine Füllkörperkolonne bei ambientem Druck destilliert. Man erhält 40 g (31,7 % der Theorie) 4,4-Dimethyl-1-hexen-5-on als klare Flüssigkeit vom Siedepunkt 151-154°C.

<u>Beispiel 2</u>

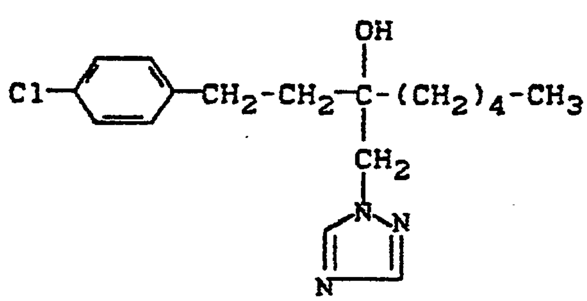

(I-2)

Eine Lösung von 89 g (0,352 Mol) 2-(4-Chlorphenylethyl)-2-pentyl-oxiran, 26,5 g (0,384 Mol) 1,2,4-Triazol, 3,5 g (0,0875 Mol) Natriumhydroxid, 1,2 ml Wasser und einer Spatelspitze α,α'-Azoisobutyronitril in 175 ml N-Methylpyrrolidon wird 4 Stunden auf 120°C erwärmt. Danach kühlt man auf Raumtemperatur ab, engt durch Abziehen des Lösungsmittels unter vermindertem Druck ein, löst den verbleibenden

Rückstand in Essigester, wäscht dreimal mit Wasser, trocknet die organische Phase über Natriumsulfat und entfernt das Lösungsmittel unter vermindertem Druck. Der Rückstand wird durch Säulenchromatographie (Kieselgel; Dichlormethan : Essigester = 1 : 1) gereinigt. Man erhält auf diese Weise 53,2 g (47,0 % der Theorie) 1-(4-Chlorphenyl)-3-(1,2,4-triazol-1-yl-methyl)-3-octanol in Form eines gelben Öls.

Die Substanz ist durch das in Abbildung 2 gezeigte Kernresonanzspektrum charakterisiert.

<u>Herstellung von Ausgangsprodukten</u>

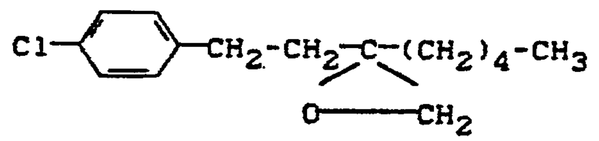

(II-2)

In eine gekühlte Lösung von 47,6 ml (0,65 Mol) Dimethylsulfid in 280 ml absolutem Dimethylsulfoxid und 150 ml absolutem Tetrahydrofuran werden 38 ml (0.61 Mol) Iodmethan eingetropft. Das Gemisch wird bei Raumtemperatur 16 Stunden gerührt. Eine Lösung von 97,5 g (0,41 Mol) 1-(4-Chlorphenyl)-3-octanon in 400 ml absolutem Toluol wird zugegeben, das Reaktionsgemisch wird auf 0 - 5°C gekühlt und innerhalb von 3 Stunden portionsweise mit 41 g (0,76 Mol) Natriummethylat versetzt. Man rührt weitere 15 Stunden bei Raumtemperatur nach, versetzt das Gemisch dann mit 1 l Wasser, trennt die organische Phase ab und extrahiert die wäßrige Phase einmal mit Toluol. Die vereinigten organischen Phasen werden zweimal mit viel Wasser gewaschen, über Natriumsulfat getrocknet und durch Abziehen des Lösungsmittels unter vermindertem Druck eingeengt. Man erhält 93,2 g eines farblosen Öles, das gemäß gaschromatographischer und massenspektrometrischer Analyse zu 84,5 % aus 2-(4-Chlorphenylethyl)-2-pentyl-oxiran besteht. Die Ausbeute errechnet sich danach zu 76,1 % der Theorie. Das Produkt wird ohne zusätzliche Reinigung für die weitere Umsetzung verwendet.

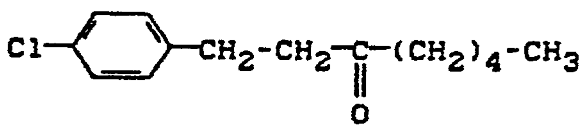

(IV-2)

Eine Lösung von 100 g (0,423 Mol) 1-(4-Chlorphenyl)-1-octen-3-on in 500 ml Toluol wird mit 15 g Raney-Nickel versetzt und im Autoklaven 5 Stunden bei 60°C unter einem Wasserstoffdruck von 70-90 bar gerührt. Danach wird das Reaktionsgemisch filtriert und unter vermindertem Druck eingeengt. Man erhält 97,3 g (96,4 % der Theorie) 1-(4-Chlorphenyl)-3-octanon in Form eines farblosen Öles.

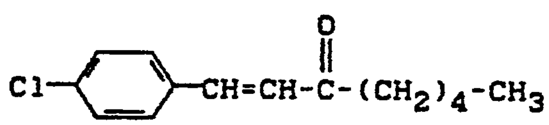

(XI-2)

Eine Lösung von 90 g (0,64 Mol) 4-Chlorbenzaldehyd und 68,5 g (0.6 Mol ) 2-Heptanon in 300 ml Ethanol wird mit 70 ml Wasser und direkt danach mit einer Lösung von 1,7 g Natriumhydroxid in 17 ml Wasser versetzt. Das Gemisch wird zunächst 1 Stunde bei Raumtemperatur gerührt, dann mit 0,6 g festem Natriumhydroxid versetzt und weitere 64 Stunden gerührt. Das ausgefallene Produkt wird abgesaugt, mit 1 l Wasser nachgewaschen, auf einem Tonteller abgedrückt und anschließend unter Vakuum über Phosphorpentoxid 16 Stunden getrocknet. Man erhält 135 g (95,1 % der Theorie) 1-(4-Chlorphenyl)-1-octen-3-on in Form eines hell-gelben Feststoffs vom Schmelzpunkt 44-47°C.

Nach der im Beispiel 2 angegebenen Methode werden auch die in der folgenden Tabelle formelmäßig aufgeführten Verbindungen hergestellt.

Tabelle

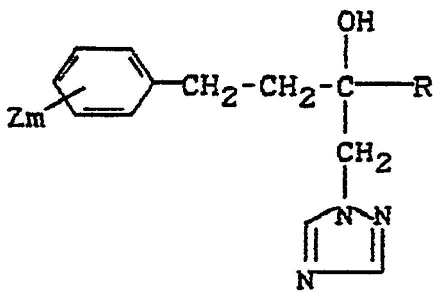

| Bsp. | Verb. Nr. | Zm | R | Charakterisierung durch |
|---|---|---|---|---|
| 3 | (I-3) | 4-Cl | $-(CH_2)_5-CH_3$ | NMR-Spektrum in Abb. 3 |
| 4 | (I-4) | 4-Cl | $-(CH_2)_6-CH_3$ | NMR-Spektrum in Abb. 4 |
| 5 | (I-5) | 4-Cl | $-CH_2-CH_2-CH(CH_3)_2$ | Schmelzpunkt 78 - 79° C |
| 6 | (I-6) | $4-CH=NOCH_3$ | $-CH_2-CH(CH_3)_2$ | NMR-Spektrum in Abb. 5 |
| 7 | (I-7) | $4-CH=NOCH_3$ | $-CH_2-CH_2-CH(CH_3)_2$ | NMR-Spektrum in Abb. 6 |
| 8 | (I-8) | 4-Cl | $-CH_2-CH(CH_3)_2$ | NMR-Spektrum in Abb. 7 |
| 9 | (I-9) | 4-Cl | 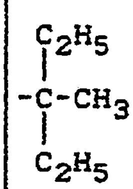 | Schmelzpunkt 112-113° C |
| 10 | I-10 | 4-F | 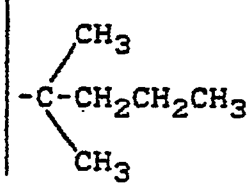 | Schmelzpunkt 52 - 55° C |

Tabelle (Fortsezung)

| Bsp. | Verb. Nr. | Zm | R | Charakterisierung durch |
|---|---|---|---|---|
| 11 | I-11 | 4-F | 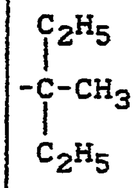 | Schmelzpunkt 83 - 84°C |
| 12 | I-12 | - | 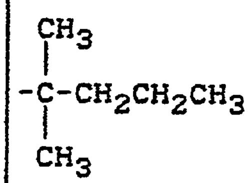 | Schmelzpunkt 84,5°C |
| 13 | I-13 | $4-OCF_3$ | 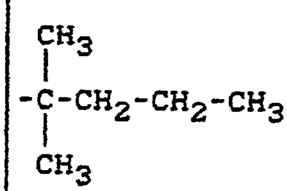 | NMR-Spektrum in Abb. 8 |
| 14 | I-14 | $4-CH_3$ | 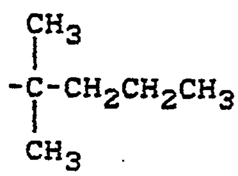 | NMR-Spektrum in Abb. 9 |

In den folgenden Verwendungsbeispielen wird die nachstehend angegebene Verbindung als Vergleichssubstanz eingesetzt:

A =

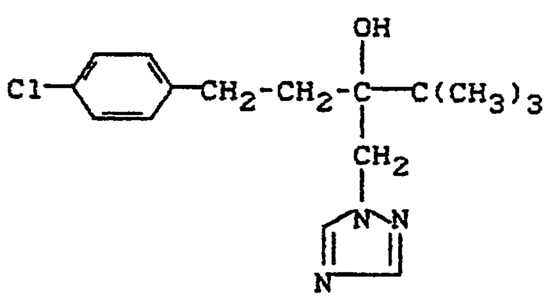

Beispiel A

Venturia-Test (Apfel) / kurativ

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Konidiensuspension des Apfelschorferregers (Venturia inaequalis) inokuliert. Die Pflanzen verbleiben 1 Tag bei 20°C und 100 % relativer Luftfeuchtigkeit in einer Inkubationskabine und werden dann im Gewächshaus aufgestellt. Nach einer angegebenen Stundenzahl werden die Pflanzen mit der Wirkstoffzubereitung tropfnaß gespritzt.

Die Pflanzen werden dann im Gewächshaus bei 20°C und einer relativen Luftfeuchtigkeit von ca. 70 % aufgestellt.

12 Tage nach der Inokulation erfolgt die Auswertung.

In diesem Test zeigen die erfindungsgemäßen Stoffe (I-1) und (I-7) eine bessere Wirkung als die Vergleichssubstanz (A).

Beispiel B

Cercospora-Test (Mungobohne) / protektiv

Lösungsmittel: 4,7 Gewichtsteil Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Cercospora canescens inokuliert und verbleiben einen Tag in einer dunklen Feuchtekammer bei 22°C und 100 % relativer Luftfeuchtigkeit.

Anschließend werden die Pflanzen unter Belichtung im Gewächshaus bei 23°C und 80 % relativer Luftfeuchtigkeit aufgestellt.

20 Tage nach der Inokulation erfolgt die Auswertung.

In diesem Test zeigen die erfindungsgemäßen Stoffe (I-7) und (I-8) eine bessere Wirksamkeit als die Vergleichssubstanz (A).

Beispiel C

Pflanzenverträglichkeitstest Testpflanze: Gurke
Versuchsdauer: 7 Tage
Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Mit dieser Wirkstoffzubereitung besprüht man junge Pflanzen taufeucht und stellt sie in einem Gewächshaus bei ca. 20°C auf.

Die Pflanzen werden auf Schäden wie Wuchsbeeinträchtigungen, Verfärbungen und Nekrosen ausgewertet. Angegeben wird der Schädigungsgrad der Pflanzen in %. Dabei bedeutet:
0 %: keine Schäden
100 %: Pflanze vollkommen geschädigt.

In diesem Test zeigen die erfindungsgemäßen Verbindungen (I-1) und (I-7) eine bessere Verträglichkeit als die Vergleichssubstanz (A).

Beispiel D

Erysiphe-Test (Gerste) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 x 12 Korn 2 cm tief in eine Standarderde. 7 Tage nach der Aussaat, wenn die jungen Pflanzen ihr erstes Blatt entfaltet haben, werden sie mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-8) eine bessere Wirkung als die Vergleichssubstanz (A).

**Patentansprüche**

1. Hydroxyalkyl-triazolyl-Derivate der Formel

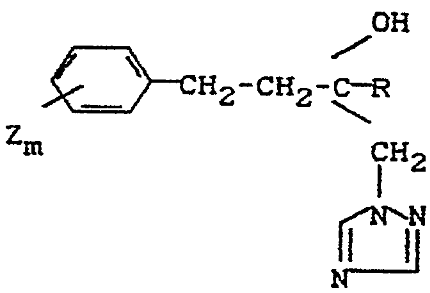

(I)

in welcher

R für die Reste der Formeln $-CH_2-CH(CH_3)_2$, $-(CH_2)_4-CH_3$, $-(CH_2)_5-CH_3$, $-(CH_2)_6-CH_3$,

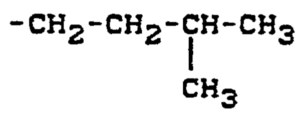,

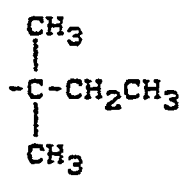, 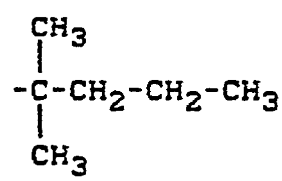 oder 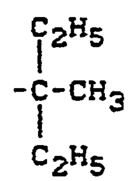 steht,

Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl oder Alkoximinomethyl mit 1 bis 4 Kohlenstoffatomen in der Alkoxygruppe steht und

m für die Zahlen 0, 1, 2 oder 3 steht,

sowie deren Säureadditions-Salze und Metallsalz-Komplexe.

2. Hydroxyalkyl-triazolyl-Derivate der Formel (I) gemäß Anspruch 1, in denen

R für die Reste der Formeln $-CH_2-CH(CH_3)_2$, $-(CH_2)_4-CH_3$, $-(CH_2)_5-CH_3$, $-(CH_2)_6-CH_3$,

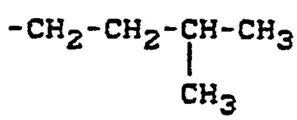,

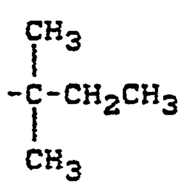, 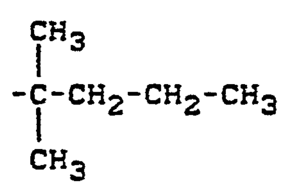 oder 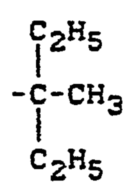 steht,

Z für Fluor, Chlor, Brom, Methyl, Ethyl, Trifluormethyl, Trifluormethoxy, Trifluormethylthio, Phenyl, Methoximinomethyl oder Ethoximinomethyl steht und
m für die Zahlen 0, 1, 2 oder 3 steht.

3. Verfahren zur Herstellung von Hydroxyalkyltriazolyl-Derivaten der Formel (I) gemäß Anspruch 1 sowie von deren Säureadditions-Salzen und Metallsalz-Komplexen, dadurch gekennzeichnet, daß man Oxirane der Formel

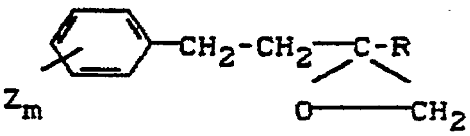

(II)

in welcher R, Z und m die oben angegebene Bedeutung haben, mit 1,2,4-Triazol der Formel

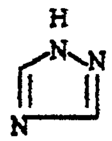

(III)

in Gegenwart eines Verdünnungsmittels und gegebenenfalls in Gegenwart eines Säurebindemittels sowie gegebenenfalls in Gegenwart eines Katalysators umsetzt und gegebenenfalls anschließend an die so erhaltenen Verbindungen der Formel (I) eine Säure oder ein Metallsalz addiert.

4. Fungizide Mittel, gekennzeichnet durch einen Gehalt an mindestens einem Hydroxyalkyl-triazolyl-Derivat der Formel (I) gemäß Anspruch 1 bzw. an einem Säureadditions-Salz oder Metallsalz-Komplex eines Hydroxyalkyl-triazolyl-Derivates der Formel (I).

5. Verfahren zur Bekämpfung von Pilzen, dadurch gekennzeichnet, daß man Hydroxyalkyl-triazolyl-Derivate der Formel (I) gemäß Anspruch 1 bzw. deren Säureadditions-Salze oder Metallsalz-Komplexe auf die Pilze und/oder deren Lebensraum ausbringt.

6. Verwendung von Hydroxyalkyl-triazolyl-Derivaten der Formel (I) gemäß Anspruch 1 bzw. von deren Säureadditions-Salzen und Metallsalz-Komplexen zur Bekämpfung von Pilzen.

7. Verfahren zur Herstellung von fungiziden Mitteln, dadurch gekennzeichnet, daß man Hydroxyalkyl-triazolyl-Derivate der Formel (I) gemäß Anspruch 1 bzw. deren Säureadditionssalze oder Metallsalz-Komplexe mit Streckmitteln und/oder oberflächenaktiven Stoffen vermischt.

8. Oxirane der Formel

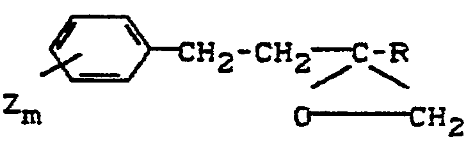

(II)

in welcher
R für die Reste der Formeln $-CH_2-CH(CH_3)_2$, $-(CH_2)_4CH_3$, $-(CH_2)_5-CH_3$, $-(CH_2)_6-CH_3$,

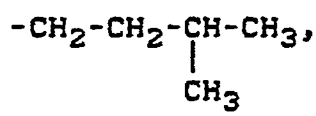

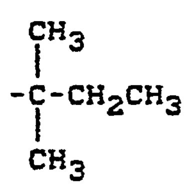 , 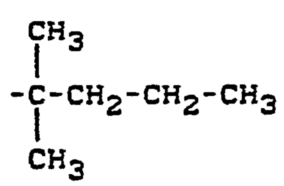 oder 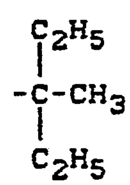 steht ,

Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl oder Alkoximinomethyl mit 1 bis 4 Kohlenstoffatomen in der Alkoxygruppe steht und
m für die Zahlen 0, 1, 2 oder 3 steht.

9. Verfahren zur Herstellung von Oxiranen der Formel (II) gemäß Anspruch1, dadurch gekennzeichnet, daß man

a) in einer ersten Stufe Ketone der Formel

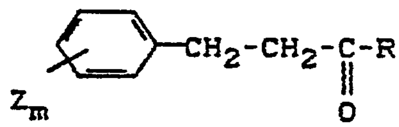

(IV)

in welcher
R, Z und m die oben angegebene Bedeutung haben,
mit Methyl-triphenyl-phosphoniumbromid der Formel

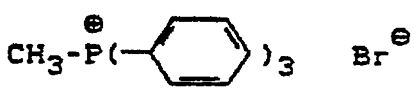

(V)

in Gegenwart einer Base und in Gegenwart eines Verdünnungsmittels umsetzt und dann in einer zweiten Stufe die so erhaltenen Verbindungen der Formel

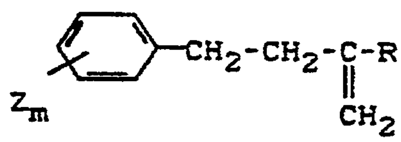

(VI)

in welcher
R, Z und m die oben angegebene Bedeutung haben,
mit Persäuren in Gegenwart eines Verdünnungsmittels umsetzt, oder

b) Ketone der Formel

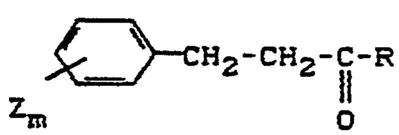

(IV)

in welcher
R, Z und m die oben angegebene Bedeutung haben,
entweder

α) mit Dimethyloxosulfonium-methylid der Formel

$$\overset{\delta^{\ominus}}{(CH_3)_2SO}\ \overset{\delta^{\ominus}}{CH_2} \qquad (VII)$$

oder

β) mit Dimethylsulfonium-methylid der Formel

$$\overset{\delta^{\ominus}}{(CH_3)_2S}\ \overset{\delta^{\ominus}}{CH_2} \qquad (VIII)$$

in Gegenwart eines Verdünnungsmittels umsetzt

10. Verbindungen der Formel

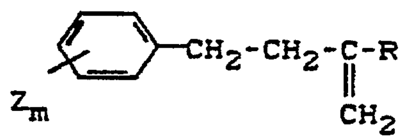 (VI)

in welcher

R für die Reste der Formeln $-CH_2-CH(CH_3)_2$, $-(CH_2)_4-CH_3$, $-(CH_2)_5-CH_3$, $-(CH_2)_6-CH_3$,

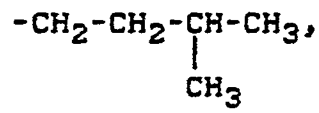

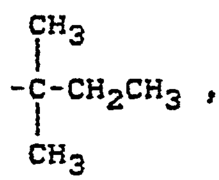 , 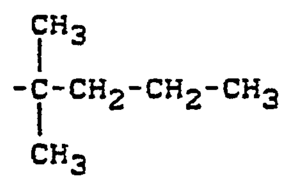 oder 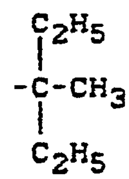 steht ,

Z für Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl oder Alkoximinomethyl mit 1 bis 4 Kohlenstoffatomen in der Alkoxygruppe steht und

m für die Zahlen 0, 1, 2 oder 3 steht.

**Claims**

1. Hydroxyalkyl-triazolyl derivatives of the formula

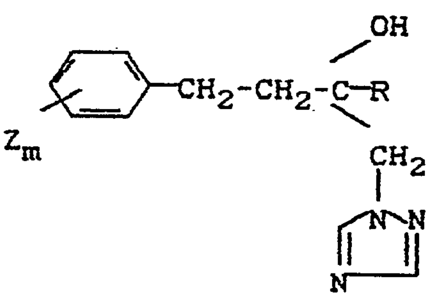 (I)

in which

R represents the radicals of the formulae $-CH_2-CH(CH_3)_2$, $-(CH_2)_4-CH_3$, $-(CH_2)_5-CH_3$, $-(CH_2)_6-CH_3$,

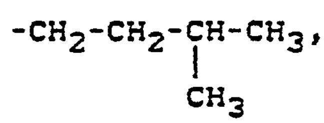

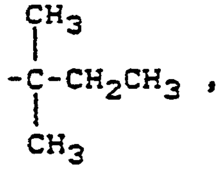 , 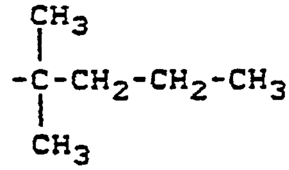 or 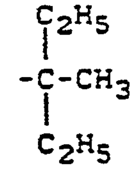

Z represents halogen, alkyl with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio

with 1 or 2 carbon atoms and 1 to 5 halogen atoms, phenyl or alkoximinomethyl with 1 to 4 carbon atoms in the alkoxy group and
m represents the numbers 0, 1, 2 or 3,
and their acid addition salts and metals salts complexes.

2. Hydroxyalkyl-triazolyl derivatives of the formula (I) according to Claim 1, in which
R represents the radicals of the formulae $-CH_2-CH(CH_3)_2$, $-(CH_2)_4-CH_3$, $-(CH_2)_5-CH_3$, $-(CH_2)_6-CH_3$,

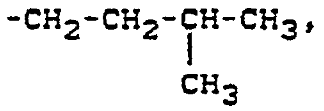

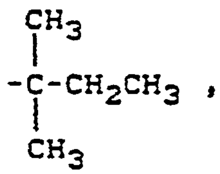

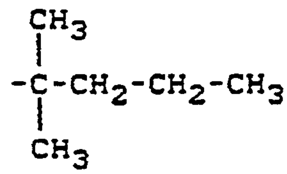

or

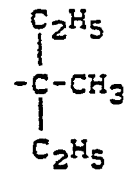

Z represents fluorine, chlorine, bromine, methyl, ethyl, trifluoromethyl, trifluoromethoxy, trifluoromethylthio, phenyl, methoximinomethyl or ethoximinomethyl and
m represents the numbers 0, 1, 2 or 3.

3. Process for the preparation of hydroxyalkyl-triazolyl derivatives of the formula (I) according to Claim 1, and of their acid addition salts and metal salt complexes, characterized in that oxiranes of the formula

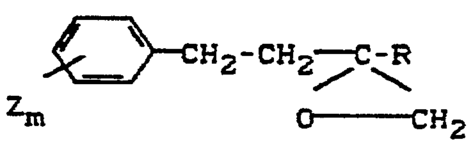

(II)

in which
R, Z and m have the abovementioned meaning are reacted with 1,2,4-triazole of the formula

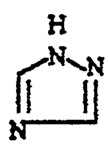

(III)

in the presence of a diluent and if appropriate in the presence of an acid binding agent and if appropriate in the presence of a catalyst and then if appropriate an acid or a metal salt is subsequently added to the compounds of the formula (I) so obtained.

4. Fungicidal agent, characterized in that it contains at least one hydroxyalkyl-triazolyl derivative of the formula (I) according to Claim 1 or an acid addition salt or metal salt complex of a hydroxyalkyltriazolyl derivative of the formula (I).

5. Process for combating fungi, characterized in that hydroxyalkyl-triazolyl derivatives of the formula (I) according to Claim 1 or their acid addition salts or metal salt complexes are applied to the fungi and/or their environment.

6. Use of hydroxyalkyl-triazolyl derivatives of the formula (I) according to Claim 1 or their acid addition salts and metal salt complexes for combating fungi.

7. Process for the preparation of fungicidal agents, characterized in that hydroxyalkyl-triazolyl derivatives of the formula (I) according to Claim 1 or their acid addition salts or metal salt complexes are mixed with extenders and/or surface-active substances.

8. Oxiranes of the formula

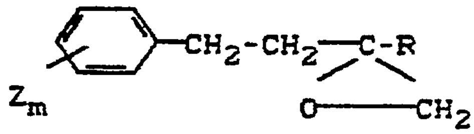 (II)

in which

R represents the radicals of the formulae $-CH_2-CH(CH_3)_2$, $-(CH_2)_4-CH_3$, $-(CH_2)_5-CH_3$, $-(CH_2)_6-CH_3$,

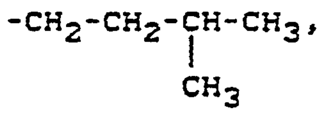

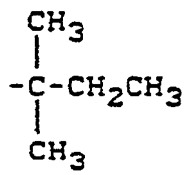 , 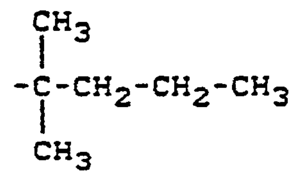 or 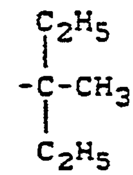

Z represents halogen, alkyl with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl or alkoximinomethyl with 1 to 4 carbon atoms in the alkoxy group and

m represents the numbers 0, 1, 2 or 3.

9. Process for the preparation of oxiranes of the formula (II) according to Claim 8, characterized in that

a) in a first step ketones of the formula

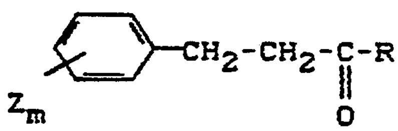 (IV)

in which R, Z and m have the abovementioned meaning, are reacted with methyl-triphenyl-phosphonium bromide of the formula

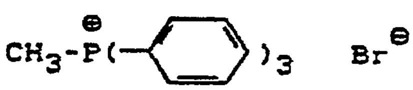 (V)

in the presence of a base and in the presence of a diluent and then in a second step the compounds so obtained of the formula

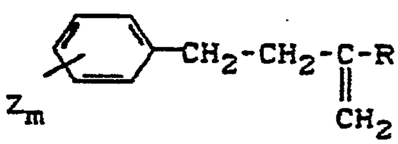 (VI)

in which R, Z and m have the abovementioned meaning, are reacted with peracids in the presence of a diluent, or

b) ketones of the formula

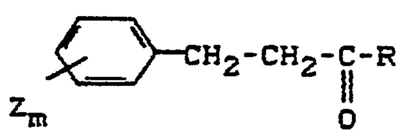 (IV)

in which

R, Z and m have the abovementioned meaning, are reacted either

α) with dimethyloxosulphonium methylide of the formula

$$\overset{\delta\ominus}{(CH_3)_2SO}\ \overset{\delta\ominus}{CH_2} \qquad (VII)$$

or

β) with dimethylsulphonium methylide of the formula

$$\overset{\delta\ominus}{(CH_3)_2S}\ \overset{\delta\ominus}{CH_2} \qquad (VIII)$$

in the presence of a diluent.

10. Compounds of the formula

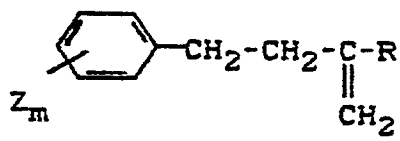

(VI)

in which

R represents the radicals of the formulae $-CH_2-CH(CH_3)_2$, $-(CH_2)_4-CH_3$, $-(CH_2)_5-CH_3$, $-(CH_2)_6-CH_3$,

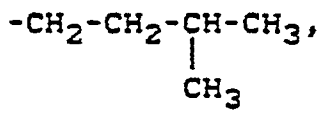

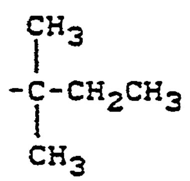 ,

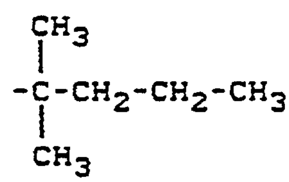

or

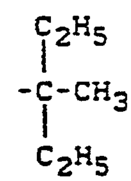

Z represents halogen, alkyl with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl or alkoximinomethyl with 1 to 4 carbon atoms in the alkoxy group and

m represents the numbers 0, 1, 2 or 3.

**Revendications**

1. Dérivés d'hydroxyalkyl-triazolyle répondant à la formule

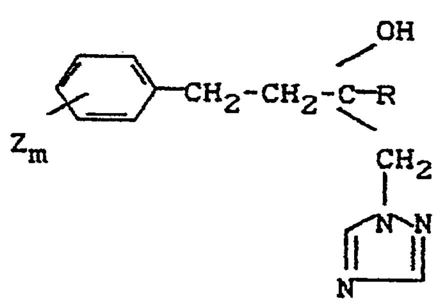

(I)

dans laquelle

R représente les radicaux répondant aux formules $CH_2$-$CH(CH_3)_2$, -$(CH_2)_4$-$CH_3$, -$(CH_2)_5$-$CH_3$, -$(CH_2)_6$-$CH_3$,

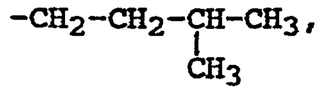

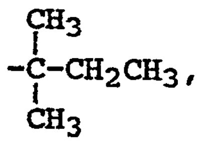 , 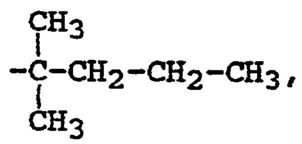 , ou 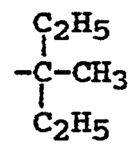 ,

Z représente un atome d'halogène, un groupe alkyle contenant 1 à 4 atomes de carbone, un groupe halogènalkyle contenant 1 ou 2 atomes de carbone et 1 à 5 atomes d'halogène, un groupe halogènalcoxy contenant 1 ou 2 atomes de carbone et 1 à 5 atomes d'halogène, un groupe halogènalkylthio contenant 1 ou 2 atomes de carbone et 1 à 5 atomes d'halogène, un groupe phényle ou un groupe alcoxyiminométhyle contenant 1 à 4 atomes de carbone dans le groupe alcoxy et
m représente les nombres 0, 1, 2 ou 3,
ainsi que leurs sels d'addition d'acides et leurs complexes de sels de métaux.

2. Dérivés d'hydroxyalkyl-triazolyle de formule (I) selon la revendication 1, dans lesquels
R représente les radicaux répondant aux formules $CH_2$-$CH(CH_3)_2$, -$(CH_2)_4$-$CH_3$, -$(CH_2)_5$-$CH_3$, $(CH_2)_6$-$CH_3$,

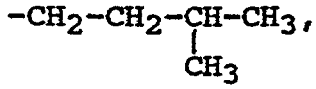

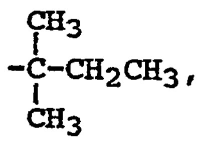 , 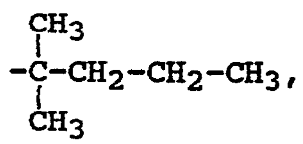 , ou 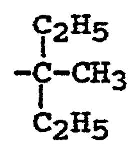 ,

Z représente un atome de fluor, un atome de chlore, un atome de brome, un groupe méthyle, un groupe éthyle, un groupe trifluorométhyle, un groupe trifluorométhoxy, un groupe trifluorométhylthio, un groupe phényle, un groupe méthoxyiminométhyle ou un groupe éthoxyiminométhyle et
m représente les nombres 0, 1, 2 ou 3.

3. Procédé de préparation de dérivés d'hydroxyalkyl-triazolyle de formule (I) selon la revendication 1, ainsi que de leurs sels d'addition d'acides et de leurs complexes de sels de métaux, caractérisé en ce qu'on fait réagir des oxirannes répondant à la formule

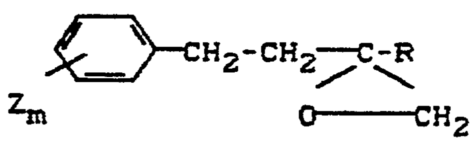 (II)

dans laquelle
R, Z et m ont la signification définie ci-dessus, avec le 1,2,4-triazole répondant à la formule

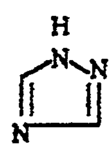 (III)

en présence d'un diluant et éventuellement en présence d'un agent fixateur d'acides, ainsi qu'éventuellement en présence d'un catalyseur et en ce qu'éventuellement on ajoute ensuite un acide ou un sel de métal aux composés de formule (I) ainsi obtenus.

4. Agents fongicides caractérises en ce qu'ils contiennent au moins un dérivé d'hydroxyalkyltriazolyle de formule (I) selon la revendication 1 ou un sel d'addition d'acides ou encore un complexe de sel de métal d'un dérivé d'hydroxyalkyl-triazolyle de formule (I).

5. Procédé destiné à lutter contre les champignons, caractérise en ce qu'on répand des dérivés d'hydroxyalkyl-triazolyle de formule (I) selon la revendication 1 ou leurs sels d'addition d'acides ou encore leurs complexes de sels de métaux, sur les champignons et/ou leur biotope.

6. Utilisation de dérivés d'hydroxyalkyl-triazolyle de formule (I) selon la revendication 1 ou de leurs sels d'addition d'acides ou encore de leurs complexes de sels de métaux pour lutter contre les champignons.

7. Procédé de préparation d'agents fongicides, caracterise en ce qu'on mélange des dérivés d'hydroxyalkyl-triazolyle de formule (I) selon la revendication 1 ou leurs sels d'addition d'acides ou encore leurs complexes de sels de métaux avec des diluants et/ou des substances tensio-actives.

8. Oxirannes de formule

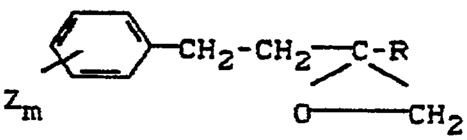 (II)

dans laquelle

R représente les radicaux répondant aux formules $CH_2-CH(CH_3)_2$, $-(CH_2)_4-CH_3$, $-(CH_2)_5-CH_3$, $-(CH_2)_6-CH_3$,

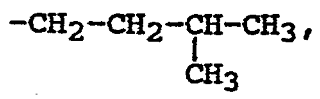

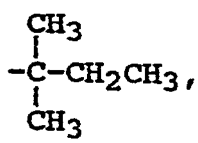, 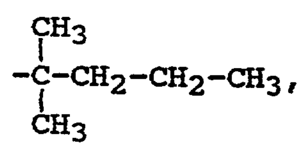, ou 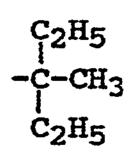,

Z représente un atome d'halogène, un groupe alkyle contenant 1 à 4 atomes de carbone, un groupe halogènalkyle contenant 1 ou 2 atomes de carbone et 1 à 5 atomes d'halogène, un groupe halogènalcoxy contenant 1 ou 2 atomes de carbone et 1 à 5 atomes d'halogène, un groupe halogènalkylthio contenant 1 ou 2 atomes de carbone et 1 à 5 atomes d'halogène, un groupe phényle ou un groupe alcoxyiminométhyle contenant 1 à 4 atomes de carbone dans le groupe alcoxy et

m représente les nombres 0, 1, 2 ou 3,

9. Procédé de préparation d'oxirannes de formule (II) selon la revendication 8, caractérisé en ce que

a) en une première étape, on fait réagir des cétones de formule

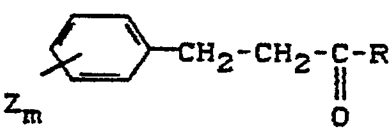 (IV)

dans laquelle R, Z et m ont la signification définie ci-dessus, avec du bromure de méthyl-triphénylphosphonium de formule

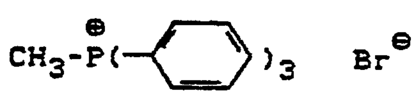 (V)

en présence d'une base et en présence d'un diluant et ensuite dans une seconde étape, on fait réagir les composés ainsi obtenus répondant à la formule

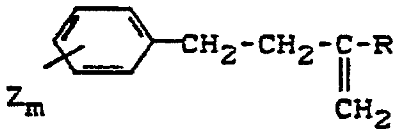 (VI)

dans laquelle R, Z et m ont la signification définie ci-dessus, avec des peracides en présence d'un diluant, ou bien

b) on fait réagir des cétones de formule

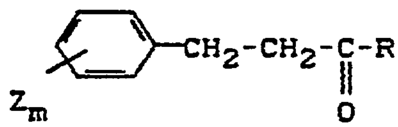 (IV)

dans laquelle R, Z et m ont la signification définie ci-dessus,

soit

α) avec du méthylure de diméthyloxosulfonium de formule

$$\overset{\delta^{\ominus}}{(CH_3)_2SO}\ \overset{\delta^{\ominus}}{CH_2} \qquad (VII)$$

soit

β) avec du méthylure de diméthylsulfonium de formule

$$\overset{\delta^{\ominus}}{(CH_3)_2S}\ \overset{\delta^{\ominus}}{CH_2} \qquad (VIII)$$

en présence d'un diluant.

10. Composés de formule

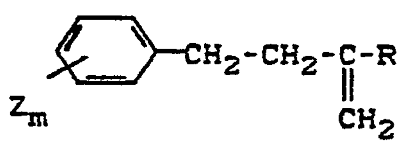 (VI)

dans laquelle

R représente les radicaux répondant aux formules $CH_2-CH(CH_3)_2$, $-(CH_2)_4-CH_3$, $-(CH_2)_5-CH_3$, $-(CH_2)_6-CH_3$,

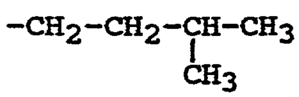 ,

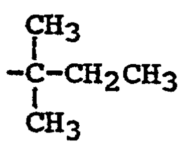 , 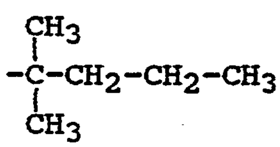 , ou 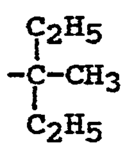 ,

Z représente un atome d'halogène, un groupe alkyle contenant 1 à 4 atomes de carbone, un groupe halogènalkyle contenant 1 ou 2 atomes de carbone et 1 à 5 atomes d'halogène, un groupe halogènalcoxy contenant 1 ou 2 atomes de carbone et 1 à 5 atomes d'halogène, un groupe halogènalkylthio contenant 1 ou 2 atomes de carbone et 1 à 5 atomes d'halogène, un groupe phényle ou un groupe alcoxyiminométhyle contenant 1 à 4 atomes de carbone dans le groupe alcoxy et

m représente les nombres 0, 1, 2 ou 3.

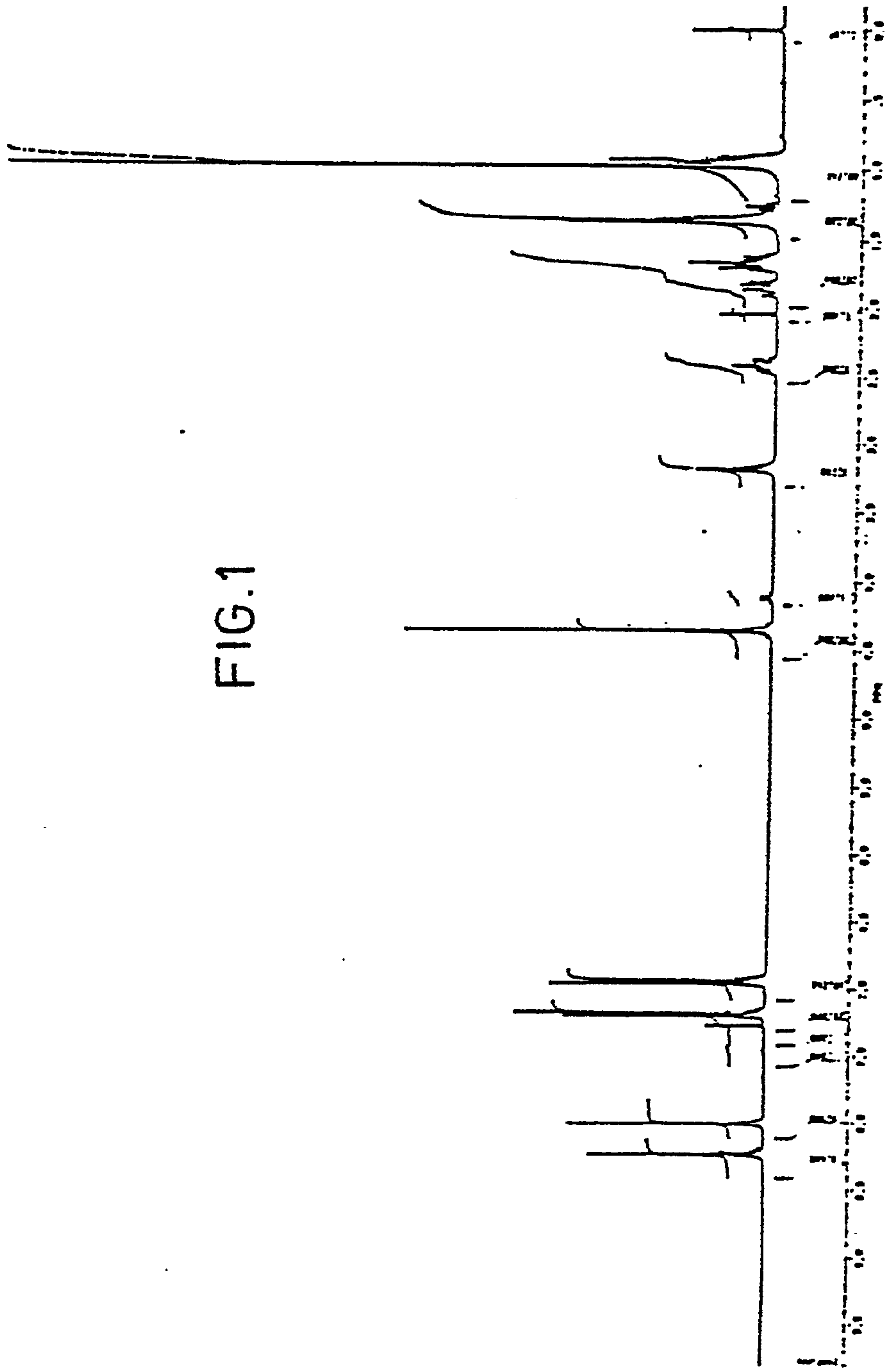

FIG.1

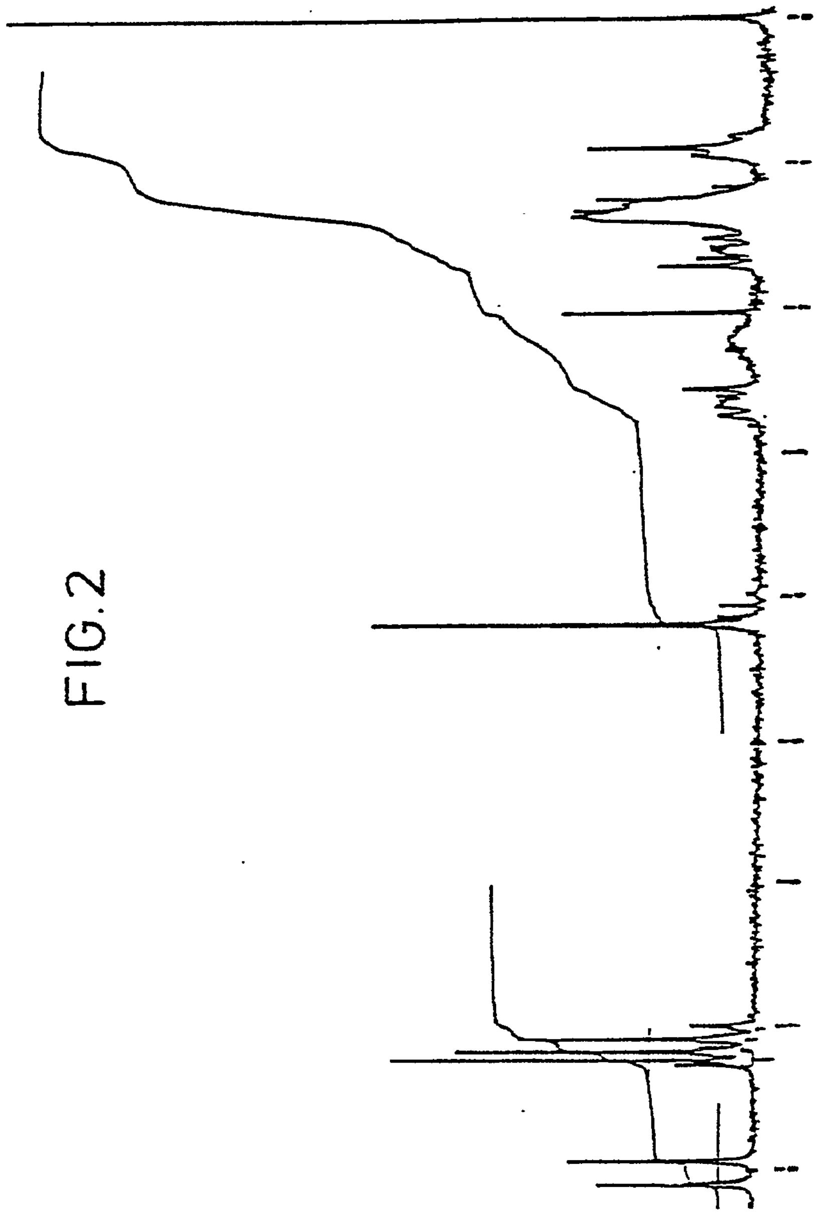

FIG. 2

FIG. 3

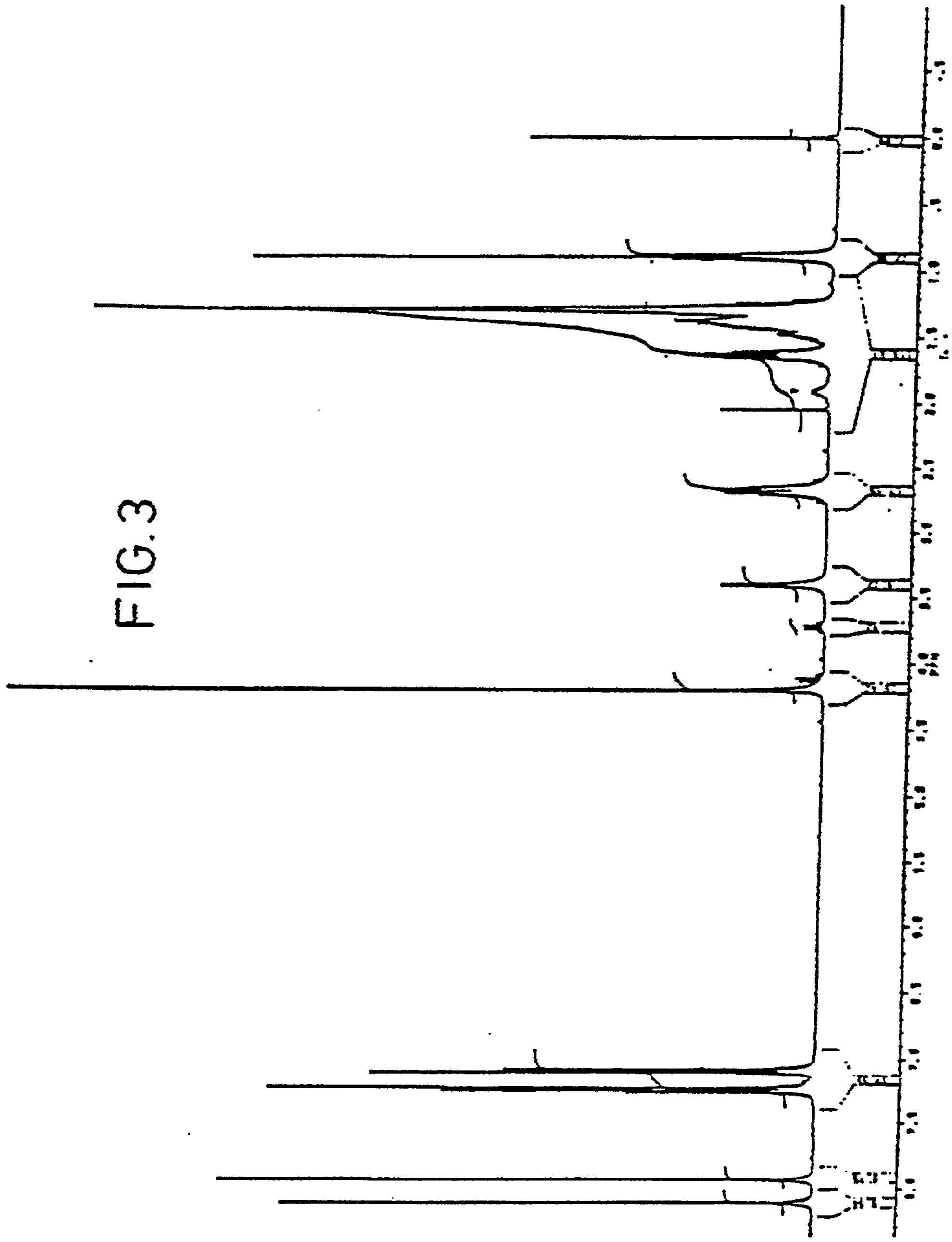

FIG. 4

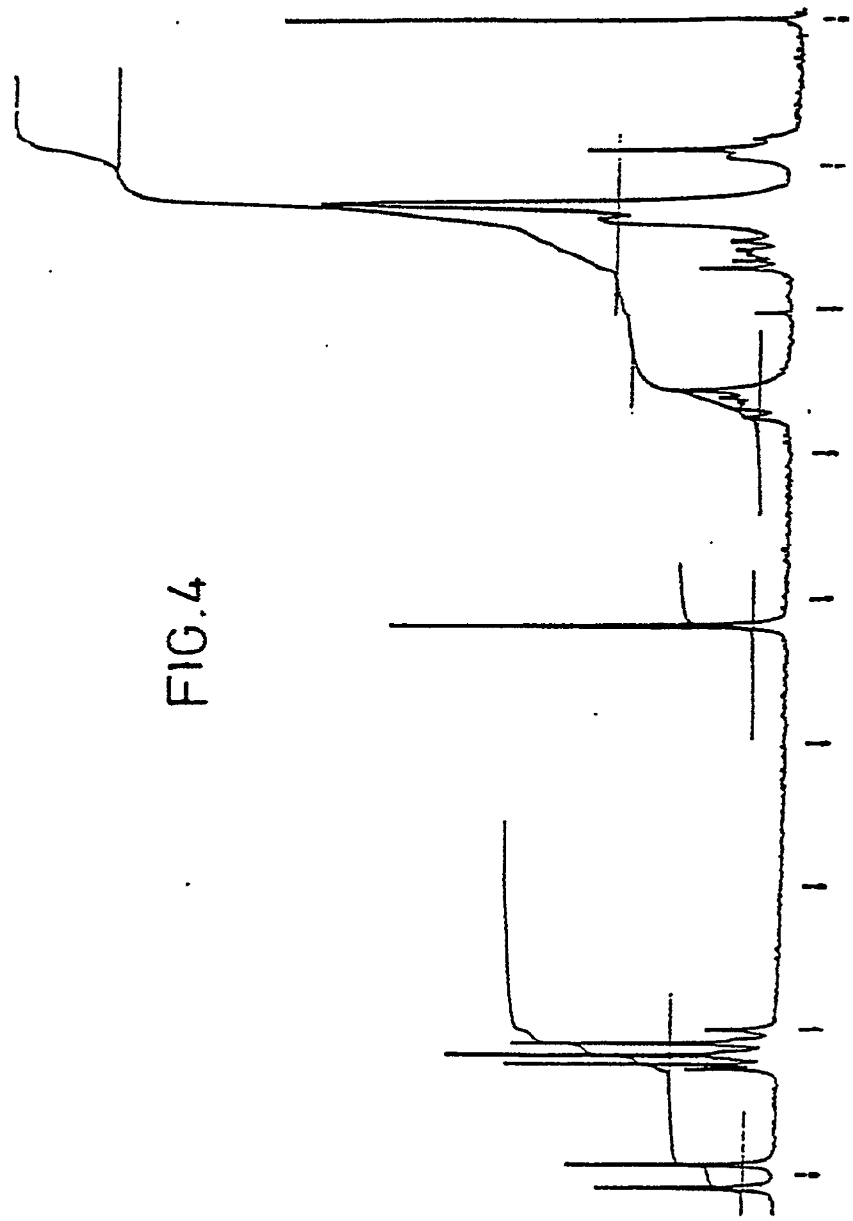

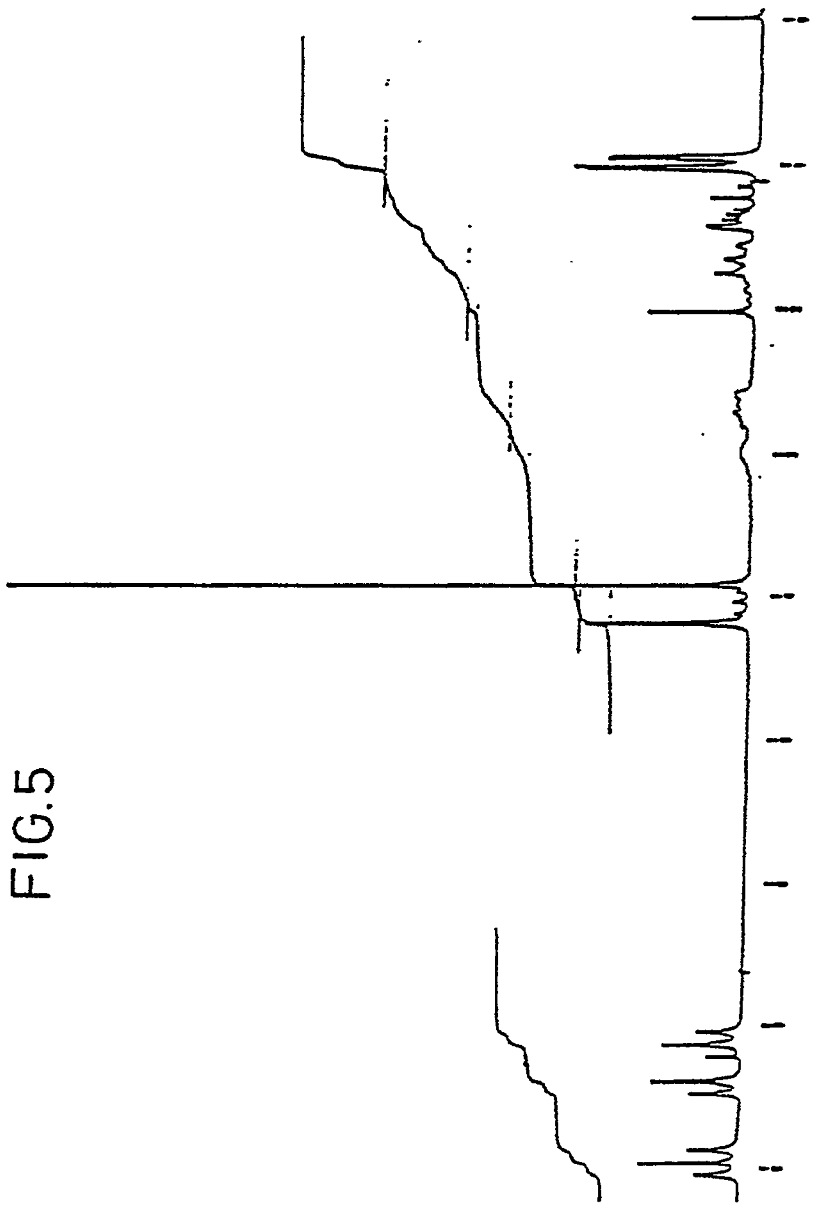

FIG.5

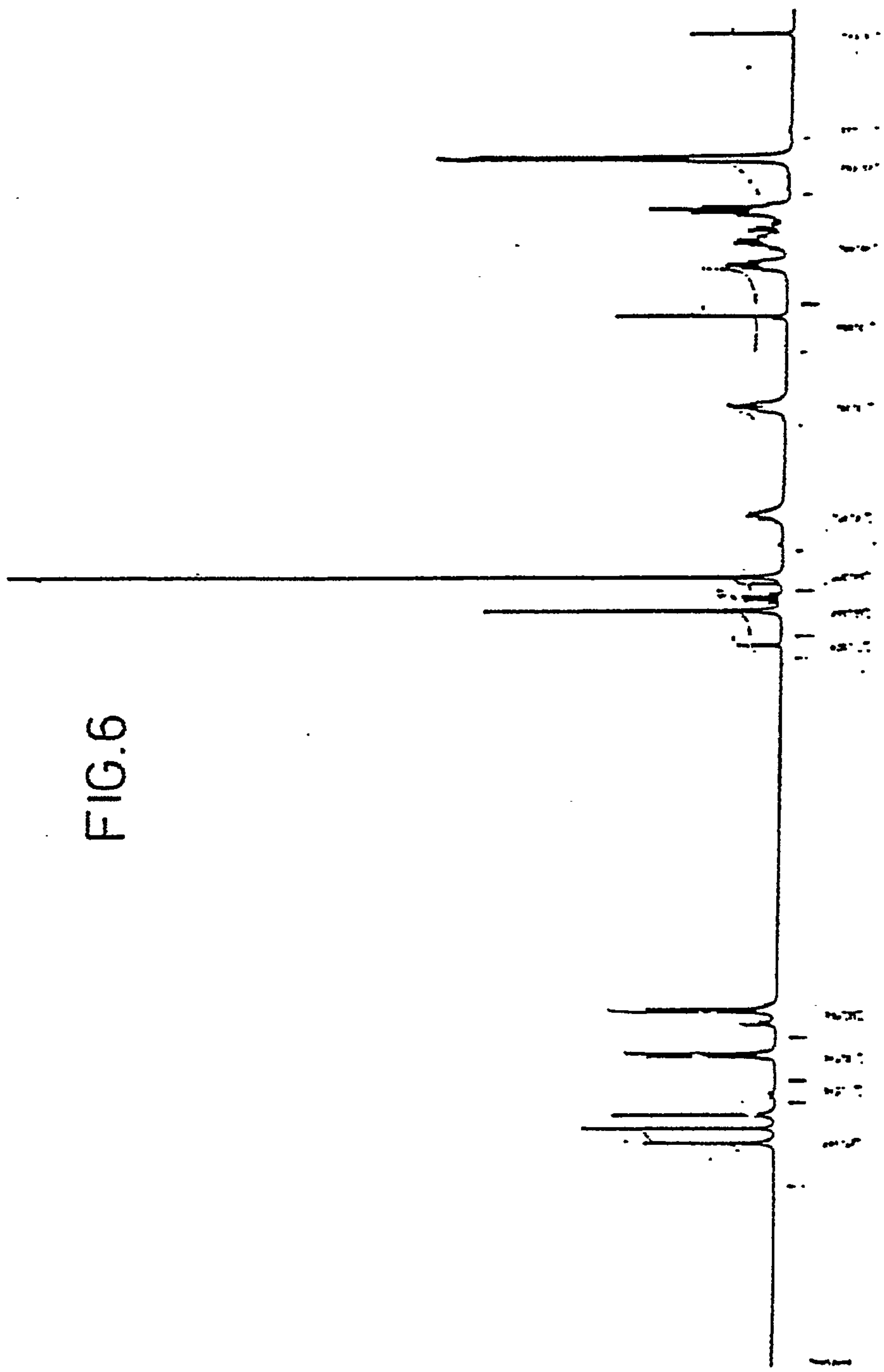

FIG.6

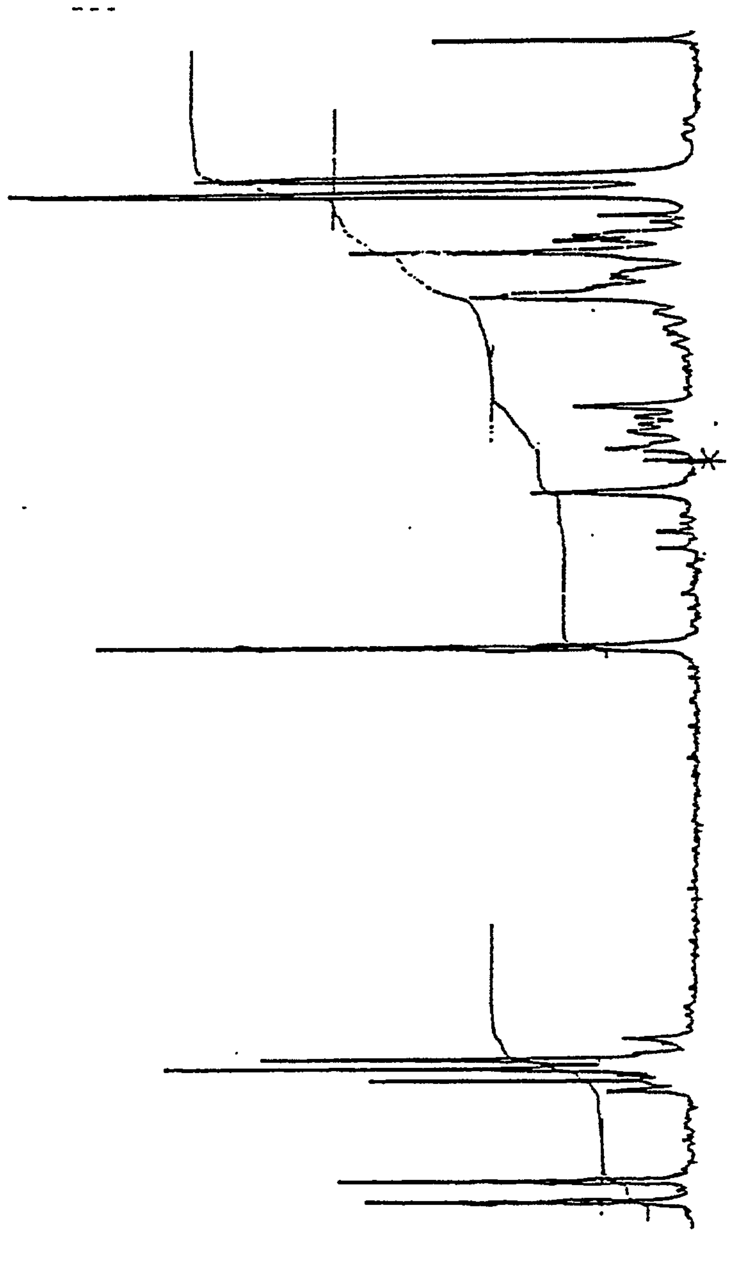

FIG. 7

FIG.8

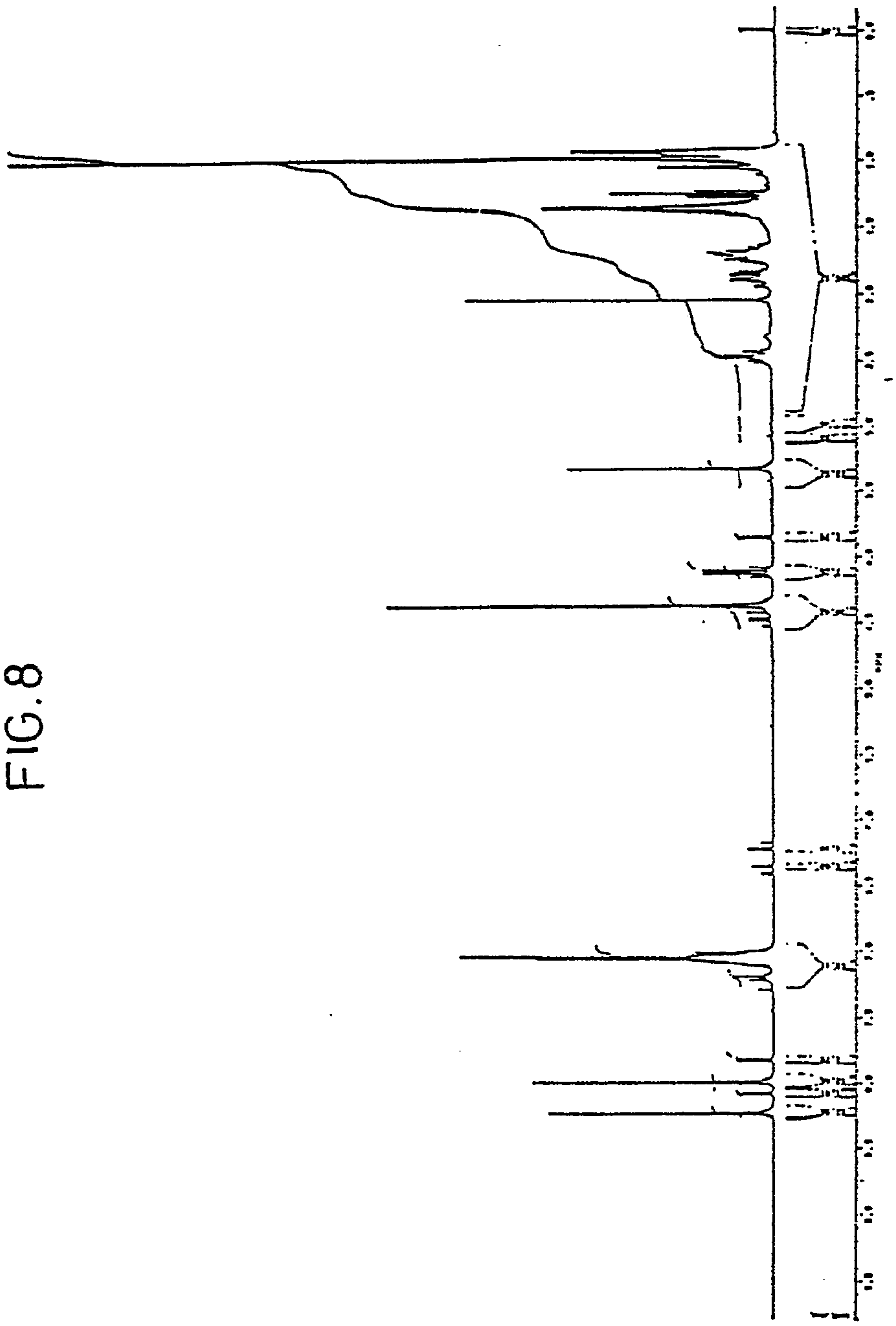

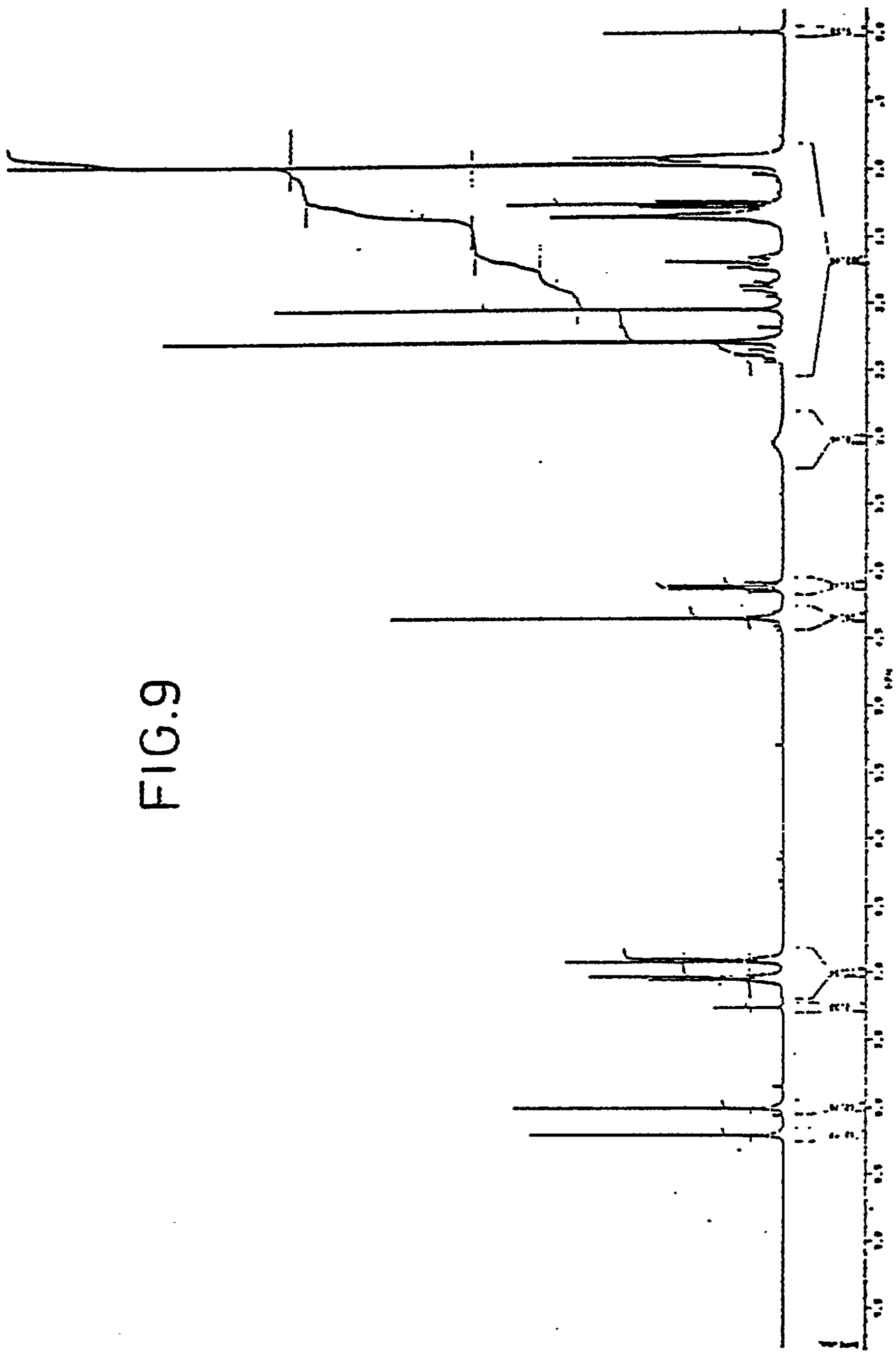

FIG.9